# EUROPÄISCHE PATENTANMELDUNG

(11) **EP 4 628 785 A2**
(43) Veröffentlichungstag der Anmeldung: **08.10.2025**
(21) Anmeldenummer: 25175214.3
(22) Anmeldetag: 15.06.2016
(51) Int. Cl.: F21V 8/00

(54) **LATERAL ABSTRAHLENDE LICHTWELLENLEITER UND VERFAHREN ZUR EINBRINGUNG VON MIKROMODIFIKATIONEN IN EINEN LICHTWELLENLEITER**

(30) Priorität: 19.06.2015 DE 102015008277; 17.11.2015 DE 102015119875
(62) Teilanmeldung aus: 16738059.1
(71) Anmelder: Clinical Laserthermia Systems GmbH, 12489 Berlin (DE)
(72) Erfinder: SCHWAGMEIER, Manuela, 12163 Berlin (DE); KNAPPE, Verena, 21465 Wentoft bei Hamburg (DE); ASHKENASI, David, 12209 Berlin (DE); CAPPIUS, Hans-Joachim, 12157 Berlin (DE)
(74) Vertreter: KIPA AB

(57) **Zusammenfassung**

Vorliegende Erfindung betrifft einen Lichtwellenleiter umfassend einen lichtwellenführenden Kern, einen Bereich des Lichtwellenleiters, wobei in dem Bereich des Lichtwellenleiters Mikromodifikationen angeordnet sind.

## Beschreibung

Die Erfindung betrifft einen Lichtwellenleiter, sowie ein Verfahren zum Einbringen von Mikromodifikationen in einen Lichtwellenleiter.

Im Stand der Technik bekannte Lichtwellenleiter bestehen in der Regel aus einem Lichtwellenleiterkern (nachfolgend als Kern benannt) und einem Lichtwellenleitermantel (nachfolgend als Mantel benannt). Dabei stellt Quarzglas ein üblicherweise verwendetes Herstellungsmaterial dar, ist jedoch nicht darauf beschränkt. Um eine verlustfreie Lichtführung innerhalb des Kerns zu gewährleisten, ist der Brechungsindex des Mantels geringer als der Brechungsindex des Kerns. Dadurch kann am Übergang zwischen dem Kern und dem Mantel die Totalreflexion ausgenutzt werden, so dass es zu einer Führung des Lichts im Kern des Lichtwellenleiters kommt. Manchmal ist der Mantel noch mit einem weiteren Cladding überzogen. Oft verfügen übliche Lichtwellenleiter zusätzlich noch über ein sogenanntes Coating und/oder einen Buffer, welche den Mantel umgeben können, Diese zusätzlichen Schichten sind üblicherweise so ausgeführt, dass diese der mechanischen Stabilität des Lichtwellenleiters im Allgemeinen dienen und insbesondere die zerstörungsfreie Biegsamkeit des Lichtwellenleiters und den mechanischen Schutz des Lichtwellenleiters vor äußeren Einflüssen gewährleisten.

Üblicherweise wird das auf einer Seite in den Kern eingekoppelte Licht innerhalb des Kerns nahezu verlustfrei fortgeführte Licht am anderen Ende des Lichtwellenleiters aus dem Lichtwellenleiter ausgekoppelt. Um den Lichtweg des Lichts innerhalb des Kerns zu ändern, ist es bekannt, Modifikationen in das Material des Kerns oder in den Randbereich zwischen Kern und Mantel einzubringen. Durch Beugung und/oder Streuung und/oder Brechung des Lichts an diesen als Störungen beschreibbaren Modifikationen kann der Lichtweg derart geändert werden, dass dadurch eine gezielte seitliche Auskopplung zumindest von Anteilen des im Kern des Lichtwellenleiters geführten Lichts erreicht werden kann. Sind die gesetzten Modifikationen über eine definierte Strecke entlang des Kerns und/oder des Randbereichs zwischen Kern und Mantel eingebracht worden, so kann die seitliche Auskopplung ebenfalls über diese definierte Strecke erfolgen.

Diese so bearbeiteten Bereiche können als sogenannte Faserapplikatoren dienen, die üblicherweise an einem Ende des Lichtwellenleiters oder auch zwischendrin ausgeführt sind. Es ist auch bekannt, diese sogenannten Faserapplikatoren im Wesentlichen durch von außen aufgesetzte Aufsätze zu realisieren. So sind z.B. wasserdicht abgeschlossene Ausätze bekannt die an einem Ende des Lichtwellenleiters auf den Lichtwellenleiter aufgesetzt sind. Dabei dient zur Änderung des Lichtweges zur seitlichen Auskopplung des Lichts entweder das mechanisch und/oder chemisch aufgeraute Ende des Lichtwellenleiters, oder eine mit Streupartikeln versetzte Flüssigkeit, die in den flüssigkeitsdicht ausgeführten Aufsätzen zirkuliert. Derartige Applikatoren sind z.B. aus den Schriften DE 41 37 983 C2, DE 42 11 526 A1 oder DE 43 16 176 A1 bekannt.

Eine weitere Bauform eines solchen aufgesetzten Applikators, wie sie z.B. aus der DE 101 29 029 A1, der US 4,660,925 oder der US 5,196,005 bekannt ist, ist ein als Hohlkörper ausgeführter Aufsatz, der an einem Ende des Lichtwellenleiters befestigt wird. Der Hohlkörper ist dabei mit einer Trägermatrix, z.B. mit einem Silikongel, gefüllt, in welche, die als Streuzentren dienenden Partikel eingebracht sind. Dabei kann die Konzentration der Streupartikel homogen verteilt oder sequentiell zum Ende hin zunehmen.

Derartige aufgesetzte Faserapplikatoren sind meist aus Polymermaterialen hergestellt und daher sehr elastisch und mechanisch belastbar, weisen aber auch entscheidende Nachteile auf, die im Wesentlichen auf der zweiteiligen Ausführung aus Lichtwellenleiter und aufgesetztem Applikator beruhen. So ist zum Beispiel die thermische Belastbarkeit von aufgesetzten Applikatoren üblicherweise wesentlich geringer als die der Lichtwellenleiter. Ebenso besteht bei derartigen Applikatoren die Gefahr der Ablösung vom Lichtwellenleiter. Üblicherweise verwenden die verschiedenen Hersteller von solchen aufgesetzten Applikatoren unterschiedliche Materialien, woraus dementsprechend unterschiedliche thermische und mechanische Eigenschaften der Applikatoren resultieren und diese nur für bestimmte Wellenlängen und Leistungen geeignet sind. Hierdurch ist ein Wechsel zwischen verschiedenen Applikatoren deutlich erschwert, zumal diese Faserapplikatoren für bestimmte Anwendungen optimiert sind. Ein weiterer entscheidender Nachteil aufgesetzter Applikatoren liegt im Fertigungsprozess. Die Montage wird üblicherweise von Hand durchgeführt und ist aufgrund der Komplexität nicht automatisiert. Zudem kann es bei der Herstellung zu einem Blasen- oder Fremdkörpereinschluss kommen, woran sich das Licht überproportional bricht und zu sogenannten unerwünschten Hotspots führt, und dadurch in einer relativ hohen Ausschussrate resultiert.

In der DE 44 07 547 Al ist ein Verfahren zur Einbringung von Modifikationen im Inneren transparenter Materialien, unter Verwendung von fokussierter Laserstrahlung beschrieben. Dabei werden mit Hilfe von Laserpulsen im Nanosekundenbereich innerhalb des transparenten Materials Mikrorisse erzeugt, die als Lichtstreuzentren dienen können. Eben diese so erzeugten Mikrorisse stellen ein Problem bei der Übertragung dieses Verfahrens auf die Anwendung bei Lichtwellenleitern dar. Denn diese Risse würden innerhalb eines Lichtwellenleiters zu einer derartigen Materialschwächung führen, die bei einer thermischen oder einer mechanischen Belastung, z.B. Biegung des Lichtwellenleiters, die Beschädigung oder sogar den Bruch des Lichtwellenleiters zur Folge haben kann. Außerdem sind diese Mikrorisse in der Größe und Ausrichtung zur Lichtwellenleiterachse nicht steuerbar.

Aus der DE 197 39 456A1 ist ein Verfahren zum Einbringen von, als Streuzentren dienenden, Mikromodifikationen in einen Lichtwellenleiter bekannt. Hierbei werden die Bearbeitungsparameter, wie z.B. die Pulsdauer der verwendeten Laserstrahlung, nicht näher beschrieben, sodass die genaue Ausprägung der entstehenden Mikromodifikationen unbestimmt ist. Die genaue Form wirkt sich allerdings entscheidend auf die Abstrahlung und mechanische Stabilität des Lichtwellenleiters aus.

Ebenso ist aus der EP1342487 B1 ein Laserapplikator bekannt, der einen Lichtwellenleiter umfasst, der Streumittel aufweist, die dazu geeignet sind, das im Inneren geführte Licht zumindest zum Teil aus dem Kern des Lichtwellenleiters herauszustreuen, wobei zumindest ein Teil der Streuzentren ein Beugungsgitter bildet. Es werden zwar Verfahren beschrieben, die geeignet sind die Streuzentren in das Innere des Lichtwellenleiters einzubringen, die genaue Einstellung der Parameter, die notwendig ist die Ausprägung der Streuzentren gezielt zu steuern wird nicht beschrieben.

In der WO 2004/005982 A2 wird ein Verfahren zur Mikrostrukturierung von Lichtwellenleitern beschrieben, in dem ultrakurze Laserpulse verwendet werden. Dieses und die weiteren oben genannten Verfahren sind allgemein formuliert und enthalten Angaben zu Form, Größe und Verteilung/Anordnung der erzeugten Mikrostrukturen ohne auf eine spezielle Gestaltung in Hinsicht auf die thermische und mechanische Stabilität einzugehen, sowohl bezüglich der Mikrostrukturen, als auch der bearbeiteten Lichtwellenleiter.

Es ist bekannt, das beim Biegen oder bei anderer Belastung (z.B. mechanisch und/oder thermisch) des Lichtwellenleiters es zu einem mechanischen Versagen (Bruch des Lichtwellenleiters) kommen kann, wenn die im Inneren des Lichtwellenleiters auftretenden Spannungen die mechanische Stabilität zu stark beeinflussen. Bereits beim Herstellungsprozess, insbesondere beim Ziehprozess, können im äußeren Bereich des Lichtwellenleitermantels Mikrorisse entstehen. Über die mechanische Verbindung zwischen dem Coating (und/oder dem Buffer) und der Mantelfläche des Mantels können die so entstehenden Spannungen abgeleitet werden, sodass es zu keiner Verstärkung (sogenannter Rissfortschritt) und damit auch nicht zu einer Beeinträchtigung der mechanischen Stabilität des Lichtwellenleiters kommt. Es ist weiterhin ein bekanntes Problem, dass jede weitergehende Bearbeitung des Inneren des Lichtwellenleiters, z.B. des Kerns oder des Mantels, die mechanische Stabilität des Lichtwellenleiters beeinträchtigt. Dadurch können Spannungen, die ein unbearbeiteter Lichtwellenleiter noch tolerieren kann, bei einem bearbeiteten Lichtwellenleiter zu einem Rissfortschritt führen, wodurch es zu einem Bruch des Lichtwellenleiters kommen kann.

Es ist Aufgabe der Erfindung, einen oder mehrere Nachteile im Stand der Technik zu vermeiden oder zu vermindern. Es ist insbesondere Aufgabe, der Erfindung einen Lichtwellenleiter bereitzustellen, der geeignet ist, zumindest Teile des im Inneren geführten Lichts seitlich abzustrahlen und zugleich über eine möglichst hohe mechanische Stabilität verfügt. Es ist weiterhin Aufgabe der Erfindung, ein Verfahren bereitzustellen zur Einbringung von Mikromodifikationen in einen Lichtwellenleiter, die dazu geeignet sind, zumindest Teile des im Inneren des Lichtwellenleiters geführten Lichts seitlich abzustrahlen.

Die Aufgabe wird ausgehend von einem Lichtwellenleiter gemäß dem Obergriff von Anspruch 1 durch die im kennzeichnenden Teil von Anspruch 1 angegebenen Merkmale, sowie durch die Merkmale des in Anspruch 11 benannte Verfahren zur Einbringung von Mikromodifikationen gelöst.

Der erfindungsgemäße Lichtwellenleiter umfasst einen lichtwellenführenden Kern, einen Bereich am distalen Ende des Lichtwellenleiters, wobei in dem Bereich im distalen Ende des Lichtwellenleiters Mikromodifikationen angeordnet sind, wobei die Anordnung der Mikromodifikationen geordnet ist. Gegenüber einer ungeordneten oder chaotischen Verteilung ist es durch eine geordnete Verteilung der Mikromodifikationen möglich, die Spannungsverteilung und die Abstrahlgeometrie im Lichtwellenleiter zu steuern. Bei einer ungeordneten Verteilung könnte es passieren, dass gerade im Bereich von Spannungsspitzen weitere Mikromodifikationen platziert sind, die die Spannung weiter erhöhen. Dies führt dann unweigerlich zu einer weiteren mechanischen Schwächung in diesem Bereich, was zu einer Beschädigung des Lichtwellenleiters führen kann. Werden die Mikromodifikationen geordnet eingebracht, so kann die Spannungsverteilung aktiv gesteuert werden. Hierdurch ist es möglich, dass die mechanische Belastung durch die Mikromodifikationen so verteilt wird, dass im Betrieb im Vergleich zu Lichtwellenleitern mit einer chaotischen Verteilung der Mikromodifikationen höhere mechanische Belastungen eingebracht werden können, bevor der Lichtwellenleiter versagt. Weiterhin kann durch eine gezielte Anordnung der Mikromodifikationen die Bearbeitungszeit gesenkt werden, da nur dort Mikromodifikationen eingebracht werden, wo sie auch sinnvoll zur seitlichen Abstrahlung des in dem Lichtwellenleiter geführten Lichtes beitragen. Bei einer ungeordneten Verteilung der Mikromodifikationen ist dies nicht gewährleistet. Daher müssen bei einer ungeordneten Verteilung der Mikromodifikationen mehr Mikromodifikationen eingebracht werden, um die gleiche seitlich abgestrahlte Intensität zu erreichen, als bei einer geordneten Anordnung der Mikromodifikationen.

In einer bevorzugten Ausführungsform des Lichtwellenleiters sind die Mikromodifikationen auf einer oder mehreren parallelen Schnittebenen angeordnet, wobei die erste Schnittebene senkrecht zur Lichtwellenleiterachse liegt und, wobei die Anordnung der Mikromodifikationen auf der ersten Schnittebene durch einen oder mehrere Parameter aus einer Gruppe von Parametern umfassend die symmetrische Anordnung der Mikromodifikationen, die Dichte der Mikromodifikationen auf der ersten Schnittebene, die Dichte der Mikromodifikationen, die Größe der Mikromodifikationen, den Abstand der Mikromodifikationen zur Lichtwellenleiterachse, den Abstand der Mikromodifikationen zueinander, der Ausrichtung der Mikromodifikationen oder anderer Parameter, mit deren Hilfe die Lage und Verteilung der Mikromodifikationen oder deren Größe oder äußere Form beschrieben wird.

In einer weiteren bevorzugten Ausführungsform des Lichtwellenleiters, wird die Anordnung der Mikromodifikationen auf einer ersten Schnittebene auf mindestens einer anderen Schnittebene wiederholt. Dies hat den Vorteil, dass Bearbeitungsroutinen wiederholt werden können und auch eine durch eine bestimmte Anordnung von Mikromodifikationen hergestellte Spannungsverteilung über einen längeren Bereich fortgeführt werden kann.

In einer weiteren besonders bevorzugten Ausführungsform des Lichtwellenleiters, ist die mindestens eine andere Schnittebene, auf der die Anordnung der Mikromodifikationen auf der ersten Schnittebene wiederholt wird, gegenüber der ersten Schnittebene um einen Winkel verdreht.

In einer weiteren besonders bevorzugten Ausführungsform des Lichtwellenleiters, ist der Abstand zwischen der ersten Schnittebene und der mindestens einer anderen Schnittebene, auf der die Anordnung der Mikromodifikationen wiederholt wird, größer als die Ausdehnung einer einzelnen Mikromodifikation.

In einer weiteren Ausgestaltung der Erfindung ist der Abstand zwischen der ersten Schnittebene und der mindestens einer anderen Schnittebene, auf der die Anordnung der Mikromodifikationen wiederholt wird, weniger als die Ausdehnung einer Mikromodifikation in axialer Richtung des Lichtwellenleiters, solange die Mikromodifikationen sich nicht überlappen oder den Strahldurchgang behindern.

In einer weiteren besonders bevorzugten Ausführungsform des Lichtwellenleiters, liegt zwischen der ersten Schnittebene und der mindestens eine andere Schnittebene, auf der die Anordnung der Mikromodifikationen der ersten Schnittebene wiederholt wird, mindestens eine weitere Schnittebene mit Mikromodifikationen, die eine andere Anordnung als die erste Schnittebene aufweist,

In einer weiteren besonders bevorzugten Ausführungsform des Lichtwellenleiters sind die Mikromodifikationen auf der ersten Schnittebene rotationssymmetrisch um die Lichtwellenleiterachse angeordnet.

In einer weiteren besonders bevorzugten Ausführungsform des Lichtwellenleiters, sind die Mikromodifikationen auf einen Hohlkegel angeordnet, wobei die Längsachse des Hohlkegels auf der Lichtwellenleiterachse liegt.

In einer weiteren besonders bevorzugten Ausführungsform des Lichtwellenleiters, sind die Mikromodifikationen auf mehreren Hohlkegeln angeordnet, wobei die Hohlkegel unterschiedliche Durchmesser aufweisen und, wobei die Längsachsen der Hohlkegel auf der Lichtwellenleiterachse liegen. Die Mikromodifikationen müssen nicht den gesamten Bereich des Kegels bis zur Spitze ausfüllen, womit u.a. abgeschnittene Kegel oder Spiralen auf einem Kegel eingeschlossen sind.

In einer weiteren besonders bevorzugten Ausführungsform des Lichtwellenleiters, der Bereich am distalen Ende des Lichtwellenleiter in Richtung der Lichtwellenleiterachse in zwei Abschnitte unterteilt ist, von denen ein erster Abschnitt dem distalen Ende des Lichtwellenleiters zugewandt ist und zweiter Abschnitt dem distalen Ende des Lichtwellenleiters abgewandt ist.

Ebenfalls eine weitere besonders bevorzugte Ausführungsform ist es, den bearbeiteten Bereich des Lichtwellenleiters in mindestens zwei Abschnitte zu unterteilen, in denen verschiedene geordnete Mikromodifikationen jeweils in verschiedenen Ausrichtungen und Ausführungsformen eingebracht sind.

Das erfindungsgemäße Verfahren zur Einbringung von Mikromodifikationen in Lichtwellenleitern umfasst, Fixierung eines Lichtwellenleiters in einer oder mehreren Halterungen, wobei der Lichtwellenleiter und/oder die Halterung beweglich gelagert sind, Fokussieren von hochenergetischer Laserstrahlung mit einer Fokussiereinrichtung in eine Fokuslage, wobei die Fokuslage im Inneren des Lichtwellenleiters positionierbar ist, wobei die Strahlung von einer Strahlungsquelle im Pulsbetrieb erzeugt wird, wobei die Fokussiereinrichtung zum Fokussieren der hochenergetischen Strahlung beweglich gelagert ist, Bewegen der Fokuslage durch den Lichtwellenleiter hindurch, wobei die Bewegung der Fokuslage im Inneren des Lichtwellenleiters gezielt in Abhängigkeit der Repetitionsrate gewählt wird, um eine vorbestimmte Anordnung der Mikromodifikationen zu erzeugen.

Bevorzugt umfasst das Verfahren zur Einbringung von Mikromodifikationen in Lichtwellenleitern die Bewegung des Lichtwellenleiters in einer Rotationsbewegung,

In einer bevorzugten Ausführungsform des Verfahrens zur Einbringung von Mikromodifikationen in Lichtwellenleitern, wird die Fokuslage kontinuierlich durch den Lichtwellenleiter bewegt.

In einer weiteren bevorzugten Ausführungsform des Verfahrens zur Einbringung von Mikromodifikationen in Lichtwellenleitern umfasst die Bewegung der Fokuslage durch den Lichtwellenleiter eine Kombination aus Rotations- und einer oder mehreren Translationsbewegungen.

In einer weiteren bevorzugten Ausführungsform des Verfahrens zur Einbringung von Mikromodifikationen in Lichtwellenleitern, ist die Bewegung der Fokuslage so mit der Repetitionsrate korreliert, dass eine geordnete gleichmäßige oder systematisch sich ändernde Anordnung von Mikromodifikationen im Lichtwellenleiter entsteht.

In einer weiteren besonders bevorzugten Ausführungsform des Verfahrens zur Einbringung von Mikromodifikationen in Lichtwellenleitern, wird die Anordnung der Mikromodifikationen durch einen oder mehrere Parameter aus einer Gruppe von Parametern umfassend die symmetrische Anordnung der Mikromodifikationen, die Dichte der Mikromodifikationen auf der Schnittebene, die Größe der Mikromodifikationen, den Abstand der Mikromodifikationen zur Lichtwellenleiterachse, den Abstand der Mikromodifikationen zueinander, der Ausrichtung der Mikromodifikationen oder anderer Parameter, mit deren Hilfe die Lage und Verteilung der Mikromodifikationen oder deren Größe oder äußere Form, beschrieben.

In einer weiteren besonders bevorzugten Ausführungsform des Verfahrens zur Einbringung von Mikromodifikationen in Lichtwellenleitern erfolgt die Einstrahlrichtung der Strahlung auf den Lichtwellenleiter unter einem Winkel zwischen Lichtwellenleiterachse und Einstrahlrichtung von ungleich 90°, in einem bevorzugten Bereich unter einem Winkel von ungleich 90° +/- 5°, in einem besonders bevorzugten Bereich unter einem Winkel von ungleich 90° +/- 10°.

In einer weiteren besonders bevorzugten Ausführungsform des Verfahrens zur Einbringung von Mikromodifikationen in Lichtwellenleitern wird die Fokussiereinrichtung zusätzlich in lateraler und transversaler Richtung in Schwingungen versetzt.

Bevorzugt kommt beim Verfahren zum Einbringen von Mikromodifikationen ein Lasersystem zum Einsatz, welches in der Lage ist, ultrakurze Laserpulse zu erzeugen. Die Pulslänge liegt dabei bevorzugt im Bereich zwischen 0,01 und 1000 ps, besonders bevorzugt im Bereich zwischen 0,05 und 10 ps, ganz besonders bevorzugt zwischen 50 und 500 fs. Die verwendeten Wellenlängen reichen vom visuellen bis in den nahen Infrarotbereich und liegen, vorzugsweise zwischen 300 und 1500 nm, besonders bevorzugt zwischen 500 - 532 nm oder 1000 - 1064 nm. Der Bereich der verwendeten Einzelpulsenergien liegt dabei bevorzugt zwischen 1 µJ und 100 µJ, besonders bevorzugt 1 bis 50 µJ. Daraus resultieren im Fokusbereich Leistungsdichten zwischen 10¹² und 10¹⁵ W/cm².

Die verwendete bzw. erreichbare Repetitionsrate des Lasersystems bestimmt entscheidend die Bearbeitungsgeschwindigkeit beim Einbringen von Mikromodifikationen in einen Lichtwellenleiter. Je höher die Repetitionsrate ist, desto schneller kann die Fokussiereinrichtung bei gleichbleibendem Abstand der Mikromodifikationen verfahren werden. Eine hohe Repetitionsrate ist also grundsätzlich zu bevorzugen. Dabei bleibt aber zu beachten, dass die Bearbeitungsachsen entsprechend schnell und präzise verfahrbar sein müssen. Des Weiteren kann es bei sehr hohen Repetitionsraten größer gleich 1 MHz zu einer Wärmeakkumulation innerhalb des Lichtwellenleiters kommen, da die in das bestrahlte Volumen des Lichtwellenleiters eingetragene Energie nicht mehr schnell genug abgeführt werden kann. Diese Wärmeakkumulation kann zu Spannungsrissen und damit zu einer mechanischen Instabilität oder sogar zur Zerstörung des Lichtwellenleiters führen. Bevorzugt wird daher eine Repetitionsrate im Bereich von 1 kHz bis 1MHz, besonders bevorzugt 1 und 100 kHz gewählt.

Die Verwendung von ultrakurzen Lichtpulsen (Pulsdauer ≤ 10 ps) hat den Vorteil, dass der Einflussbereich der eingetragenen Wärmeenergie sehr gering bleibt, wodurch die Einbringung räumlich begrenzter Mikromodifikationen ohne Schädigung des umliegenden Materials ermöglicht wird. Werden Laserpulse im Nanosekundenbereich verwendet, wird die Energie während eines einzelnen Pulses an das lonengitter des bestrahlten Materials abgegeben. Dies führt dann ebenso wie bei einer zu großen Repetitionsrate zu einer Wärmeakkumulation im bestrahlten Materialvolumen und zur Ausbildung mikroskopischer Spannungsrisse, deren Ausdehnung durchaus im Millimeterbereich liegen kann. Diese Schädigungen am Material des Lichtwellenleiters können zur Einschränkung der mechanischen Stabilität führen, bis hin zum möglichen Bruch des Lichtwellenleiters unter mechanischer und/oder thermischer Beanspruchung.

Die Verwendung von ultrakurzen Laserpulsen erlaubt eine gezielte Veränderung der Materialeigenschaften lediglich im bestrahlten Bereich von wenigen Mikrometern, ohne dabei umliegende Bereiche ungewollt zu schädigen. Die Pulsdauer reicht in diesem Fall nicht aus, um die Energie an das lonengitter des umliegenden Materials abzugeben, sodass es zu keiner bzw. zu einer stark verringerten Wärmeakkumulation kommt. So lassen sich sehr kleine Strukturen bei sehr geringen Spannungen im umliegenden Material erzeugen. Sowohl die möglichst kleinen Strukturen als auch die möglichst geringen Spannungen, sind notwendige Bedingungen zum gezielten Einbringen von Mikromodifikationen in Lichtwellenleiter. Nur so ist die gezielte Strukturierung des Lichtwellenleitermaterials möglich, ohne die mechanische Stabilität des Lichtwellenleiters zu gewährleisten.

Für das Verfahren zur Einbringung von Mikromodifikationen in Lichtwellenleiter ist eine Vorrichtung vorgesehen, die ein Achsen- und Motorsystem umfasst. Diese Vorrichtung dient einerseits zur Halterung des Lichtwellenleiters und andererseits erlaubt die Vorrichtung den Lichtwellenleiter gezielt zu verfahren, zu drehen und ermöglicht eine beliebige Positionierung des Fokus des Lasersystems innerhalb des Lichtwellenleiters. Das erfindungsgemäße Verfahren und die dazugehörige erfindungsgemäße Vorrichtung erlaubt die Form, die Verteilung und die Lage der Mikromodifikationen innerhalb des Lichtwellenleiters beliebig zu variieren. So kann z.B. die Lage bzw. die Form der Mikromodifikationen durch die Verfahrgeschwindigkeit der Linear- und Rotationachsen bzw. durch das Variieren der Repetitionsrate gezielt beeinflusst werden, Auch ist die Verteilung der Mikromodifikationen gezielt steuerbar, z.B. durch sogenanntes laserinternes "pulse picking" oder einen programmierbaren Shutter. Die Tiefe der Mikromodifikationen relativ zur Oberfläche des Lichtwellenleitermantels kann durch eine gezielte Bewegung des Fokus, durch gezielte Einstellung der Fokussiereinrichtung, beeinflusst werden. Des Weiteren ist auch die Tiefenausdehnung der einzelnen Mikromodifikationen, durch eine entsprechende Wahl von Einzelpulsenergie, Pulsdauer, Pulszahl (Einzel-, Doppel-, Mehrfachpuls), räumlicher und/oder zeitlicher Abstand, oder auch durch "pulse tailoring" gezielt beeinflussbar. Es ist ebenfalls möglich, Mikromodifikationen auf der dem Lasersystem abgewandten Seite in den Lichtwellenleiter, durch Fokussierung durch die Mitte des Lichtwellenleiters hindurch, einzubringen. Hier ist es möglich linsenartige Effekte der gebogenen Oberfläche des Lichtwellenleiters zusätzlich für die Fokussierung auszunutzen, um so Mikromodifikationen zu erzeugen, die eine noch kürzere Tiefenausdehnung besitzen. Auch die Fokuslage, bzw. Positionierung der Fokussiereinrichtung relativ zur Lichtwellenleiterachse beeinflusst die Anordnung der Mikromodifikationen. So kann durch eine Verschiebung der Einstrahlposition aus dem Lot zur Lichtwellenleiterachse die Ausrichtung der Mikromodifikationen gesteuert werden.

Jegliche Parameter, mit denen die Mikromodifikationen beschrieben werden können, wie z.B. die Tiefenposition, die räumliche Ausdehnung, die Verteilung, der Abstand zueinander, die Lage, die Ausrichtung oder auch die Form der Mikromodifikationen, können einen Einfluss auf die mechanische Stabilität des Lichtwellenleiters haben. Je kleiner die räumliche Ausdehnung der Mikromodifikationen und je größer der Abstand der Mikromodifikationen zueinander, desto geringer ist der Einfluss auf die mechanische Stabilität der Lichtwellenleiter. Andererseits ist es aber auch so, dass die Stärke der Lichtauskopplung, die durch die Mikromodifikationen bewirkt wird, sich im Wesentlichen entgegengesetzt zu den Auswirkungen auf die mechanische Stabilität verhält. Somit ist es essentiell, einen Kompromiss zwischen der ausgekoppelten Lichtintensität und der mechanischen bzw. thermischen Stabilität des Lichtwellenleiters zu finden.

In Bezug auf die Verteilung der Mikromodifikationen sind verschiedene Ausführungen denkbar. So ist es z.B. möglich, eine absichtlich unregelmäßige Verteilung der Mikromodifikationen herzustellen, um Gittereffekte, wie z.B. Interferenzen, zu vermeiden und gleichzeitig eine gleichmäßige Verteilung des ausgekoppelten Lichtes zu gewährleisten. Andererseits ist auch eine gezielte reguläre bzw. periodische Verteilung der Mikromodifikationen, wie z.B. Bragg-Gitter oder auch mehrdimensionale photonische Strukturen, denkbar. Des Weiteren besteht die Möglichkeit, die Mikromodifikationen im Innern des Lichtwellenleiters so dicht zu setzen, dass diese Modifikationen eine Linie bilden, die selbst Wellenleitereigenschaften besitzt. Diese Linienstruktur kann beliebig lang ausgeführt sein und ihr Verlauf relativ zum Lichtwellenleiter kann ebenfalls beliebig ausgeführt sein, so ist z.B. eine geradlinig oder spiralförmige oder ein helixartige Ausführung denkbar.

Die beschriebenen Bearbeitungsmöglichkeiten sind nicht auf einen bestimmten Typ eines Lichtwellenleiters beschränkt. Durch eine geeignete Anpassung der Bearbeitungsparameter ist es möglich, beliebige Typen von Lichtwellenleiter, wie z.B. Hohlfasern, Gradientenindexfasern, neuartige High-Tech Glasfasern ohne Blei, photonische Kristalle, bzw. photonische Kristallfasern zu bearbeiten.

Die Erfindung wird nachfolgend anhand mehrerer Ausführungsbeispiele näher erläutert. Es zeigen:
- Figur 1: Schematischer Aufbau eines Lichtwellenleiters mit durch Laserstrahlung induzierten Mikromodifikationen
- Figur 2: Schematische Darstellung eines Lichtwellenleiters und der Einkopplung von fokussiertem Laserlicht sowie die Möglichkeiten der Relativbewegung zwischen fokussiertem Laserlicht und Lichtwellenleiter
- Figur 3: Schematischer Aufbau der Bearbeitungsvorrichtung zur Bearbeitung von Lichtwellenleitern
a) Frontansicht
b) Seitenansicht
- Figur 4: Verfahren zur Bearbeitung von Lichtwellenleitern mit Laserstrahlung
- Figur 5: Schematischer Aufbau eines Lichtwellenleiters mit durch Laserstrahlung induzierten Mikromodifikationen
a) Lichtwellenleiter
b) Querschnitte entlang den Schnittlinien A - A, B - B, C - C, D - D und E - E
- Figur 6: Schematischer Aufbau eines Lichtwellenleiters mit durch Laserstrahlung induzierten Mikromodifikationen
a) Lichtwellenleiter
b) Querschnitte entlang den Schnittlinien A - A, B - B, C - C, D - D und E - E
- Figur 7: Schematischer Aufbau eines Lichtwellenleiters mit durch Laserstrahlung induzierten Mikromodifikationen
a) Lichtwellenleiter
b) Querschnitte entlang den Schnittlinien A - A, B - B, C - C, D - D und E - E
- Figur 8: Schematischer Aufbau eines Lichtwellenleiters mit durch Laserstrahlung induzierten Mikromodifikationen
a) - e) Querschnitte entlang den Schnittlinien A - A, B - B, C - C, D - D und E - E
f) Querschnitt entlang der Lichtwellenleiterachse
- Figur 9: Schematischer Aufbau eines Lichtwellenleiters mit durch Laserstrahlung induzierten Mikromodifikationen
a) - c) verschiedene periodische Abfolgen
- Figur 10: Schematischer Aufbau eines Lichtwellenleiters mit durch Laserstrahlung induzierten Mikromodifikationen
a) Abfolge von Querschnitten mit unterschiedlicher Verteilung und/oder Anordnung von Mikromodifikationen
b) Periodische Abfolge von Bereichen mit gleicher Abfolge von Querschnitten mit unterschiedlicher Verteilung und/oder Anordnung von Mikromodifikationen

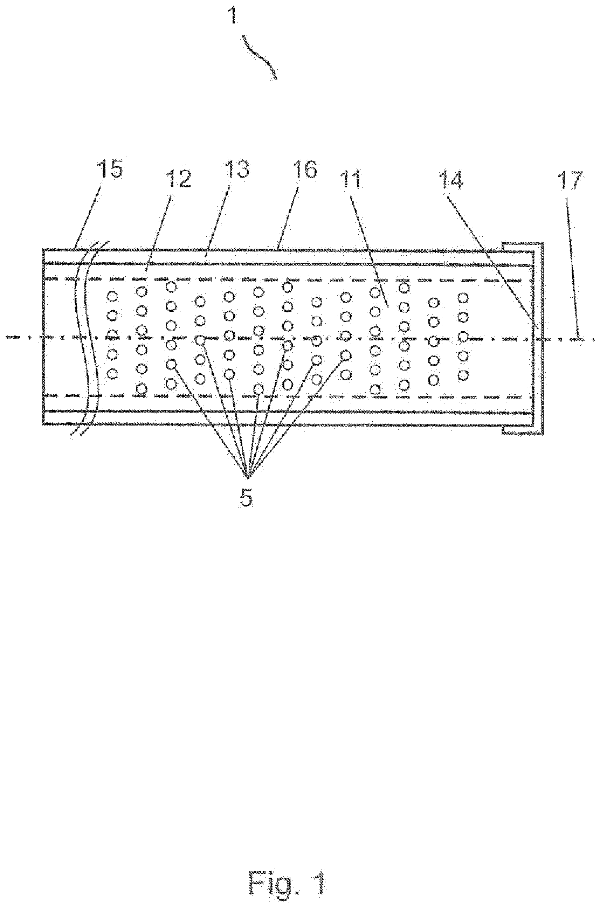

Figur 1 zeigt eine schematische Darstellung der zu bearbeitenden Lichtwellenleiter (1). Der Lichtwellenleiter umfasst einen ersten Bereich (15), der weitgehend frei von Mikromodifikationen (5) ist, und einen zweiten Bereich (16) des Lichtwellenleiters (1), in dem Mikromodifikationen (5) eingebracht sind. Dieser Bereich (16) ist zumeist am distalen Ende des Lichtwellenleiters (1) angeordnet. Optional kann der Lichtwellenleiter mit einer Endkappe (14) versehen sein, die verhindert, dass Licht aus dem Endbereich des Lichtwellenleiters (1) austritt. Diese Endkappe (14) kann durch eine Spiegelung der Lichtwellen diese erneut einer seitlichen Auskopplung durch Mikromodifikationen zuführen. Die Endkappe (14) kann durch eine geeignete, direkte Verspiegelung der Faserendfläche, die ebenfalls erfindungsgemäß ist, ersetzt werden. Der Kern (11) ist umgeben vom Mantel (12), gefolgt vom Coating und/oder Buffer (13). Kern (11) und Mantel (12) bestehen üblicherweise aus Quarz und sind unterschiedlich dotiert. Der Brechungsindex des Mantelmaterials ist geringer als der des Kernmaterials, auf diese Weise kann das Licht durch Totalreflexion am Kern-Mantel-Übergang im Lichtwellenleiter (1) transportiert werden. Der Mantel (12) ist von einem sogenannten Coating und/oder Buffer (13) umgeben, das die Spannungen beim Biegen des Lichtwellenleiters (1) aufnimmt und somit die zerstörungsfreie Biegsamkeit gewährleistet und ebenfalls dem Schutz vor mechanischen Einwirkungen auf die darunter liegenden Schichten dient. Für die Bearbeitung der Lichtwellenleiter (1) kann der für die gewählte Laserwellenlänge nicht-transparente Buffer (13) entfernt werden, so dass das Laserlicht nur noch durch den Mantel (12) fokussiert werden muss. Bei für die gewählte Laserwellenlänge transparentem Buffermaterial, z.B. Nylon oder PTFE, kann der Lichtwellenleiter (1) auch durch den Buffer (13) hindurch bearbeitet werden. Dies hat den Vorteil, dass der bearbeitete Bereich des Lichtwellenleiters (1) im Wesentlichen die gleiche erhöhte Biegefestigkeit aufweist, wie der Rest des Lichtwellenleiters (1).

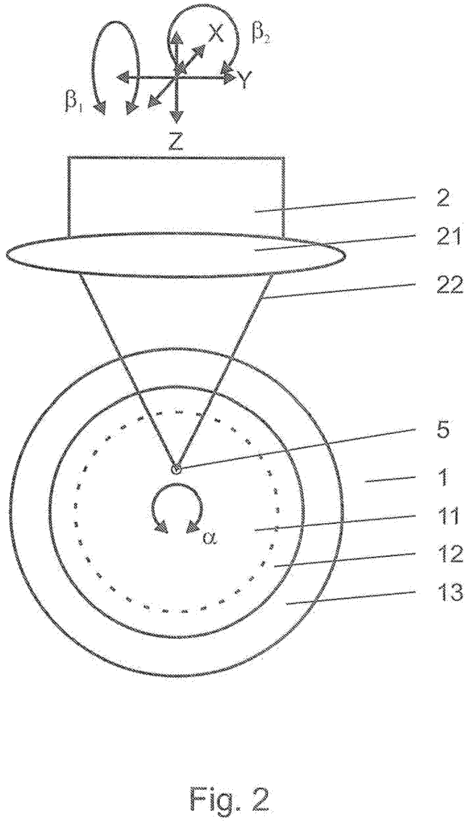

In Figur 2 ist gleichermaßen das Prinzip der Einkopplung des fokussierten Laserlichtes (2) in den Lichtwellenleiter (1) zur Einbringung der Mikromodifikationen (5) dargestellt. Figur 2 zeigt die zur Fokussierung der Laserpulse in den Kernbereich des Lichtwellenleiters (1) benötigte Fokussieroptik (21) und die erzeugten Mikromodifikationen (5), Wichtige Linsenparameter für die Einbringung der Mikromodifikationen (5) in die Lichtwellenleiter (1) sind dabei die Brennweite und die numerische Apertur (NA) der symbolisch dargestellten Fokussieroptik (21). Die Brennweite wird so kurz wie möglich gewählt, da so die Größe des Fokuspunktes minimiert werden kann. Dabei muss die Brennweite aber lang genug sein, um durch den Lichtwellenleitermantel in den Kern (11) fokussieren zu können. In einer bevorzugten Variante liegt die Brennweite der Fokussieroptik (21) zwischen 1 und 5 mm. Aber auch der Einsatz von "long-distance" Mikroskop-Objektiven mit einem Arbeitsabstand von größer 5 mm ist eine bevorzugte Umsetzungsoption. Auch eine möglichst große NA der Fokussieroptik (21) ist vorteilhaft, da diese den Öffnungswinkel der Fokussieroptik (21) bestimmt. Je größer der Öffnungswinkel, desto kürzer ist der Fokusbereich. Dies ist von großer Bedeutung, da so die Tiefenausdehnung der eingebrachten Modifikationen (5) minimiert werden kann. Der größere Öffnungswinkel führt zu einer höheren Strahldivergenz und dadurch zu einem schnell zunehmenden Strahldurchmesser vor und hinter dem Fokuspunkt. Dies reduziert die Energiedichte in den Bereichen vor und hinter dem Fokus und somit auch die Absorption und die Gefahr einer Schädigung außerhalb des Fokusbereiches.

In einer besonders bevorzugten Variante kommt als Fokussieroptik (21) eine kurzbrennweitige (f < 3,1 mm) asphärische Linse mit einer numerischen Apertur von NA > 0,68 zum Einsatz. In einer weiteren Ausführungsform kommt ein spezielles Objektiv (Linsensystem) mit einer hohen NA zum Einsatz. Dieses ist so konstruiert, dass die Wellenfronten der fokussierten Laserstrahlung (22) den gleichen Krümmungsradius besitzen wie die Materialoberfläche, auf die sie treffen. Dies hat den Vorteil, dass beim Durchgang durch die Lichtwellenleiteroberfläche die Wellenfronten nicht verzerrt werden (wave front distortion), was wiederum zu einer deutlich besseren Fokussierbarkeit im Material der Lichtwellenleiter (1) führt.

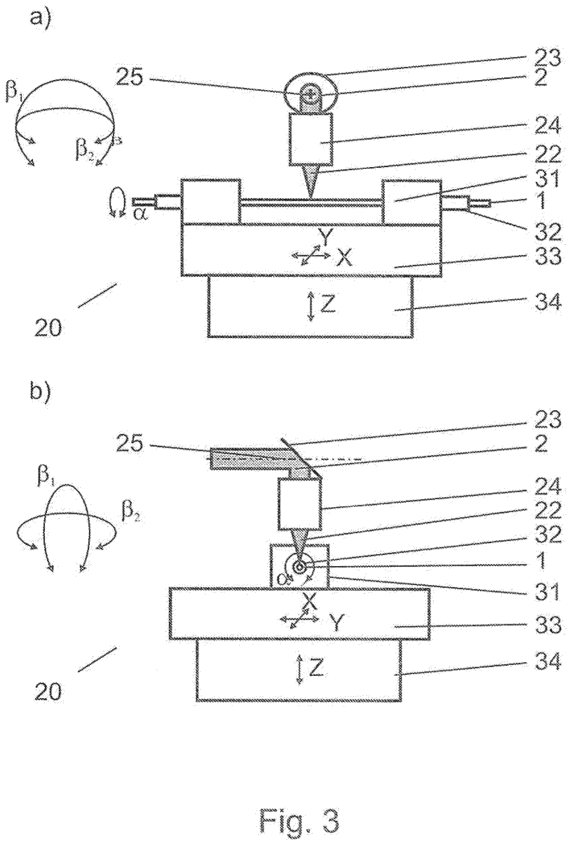

Figur 3 zeigt eine Prinzipskizze der erfindungsgemäßen Vorrichtung zur Einbringung von Mikromodifikationen in Lichtwellenleitern (20). Die Vorrichtung (20) umfasst verschiedene motorisierte Verstellvorrichtungen (33, 34) zur Ausführung einer Linearbewegung zwischen Lichtwellenleiter (1) und Fokus des fokussierten Laserstrahls (22). Die Bewegung erfolgt bevorzugt über Linearmotoren (33, 34) in den Raumrichtungen (X, Y, Z). Weiterhin umfasst die Vorrichtung (20) den Aufbau zur Einkopplung (23) des Laserlichtes (2) in die Fokussieroptik (24). Weiterhin umfasst die Vorrichtung (20) die Halterung (32) für den Lichtwellenleiter (1) und die Drehachsen (α, β₁, β₂, β₃) zur Rotation desselben. Im Unterschied zu den vorbekannten Lösungen wird die Fokussierlinse nicht bewegt, sondern ausschließlich die Lichtwellenleiter (1). Dies hat zum Vorteil, dass während der Bearbeitung keine Umlenkspiegel im Strahlengang verfahren bzw. bewegt werden müssen. Als Folge ist der Einrichtungs- bzw. Justageaufwand für die Vorrichtung deutlich geringer und gleichzeitig ergibt sich eine bessere Langzeitstabilität des Aufbaus, da alle optischen Elemente im Strahlengang fest verbaut werden können. Bereits geringfügige Ungenauigkeiten bzw. Abweichungen im Strahlengang würden bei z.B. translatorisch bewegten Umlenkspiegeln zu einer Wanderung des Laserstrahles (2) auf der Fokussieroptik (24) führen. Als Folge wandert der Fokuspunkt sowohl in der X-Y-Ebene als auch aufgrund des relativ zur Lichtwellenleiter (1) geneigten (nicht lotrechten) Strahldurchgangs durch die Fokussieroptik (24) in ZRichtung. Ein langzeitstabiler Aufbau mit reproduzierbaren Bearbeitungsergebnissen ist so nur sehr schwer oder gar nicht zu realisieren.

Die Z-Achse (34) trägt den weiteren Bearbeitungsaufbau bestehend aus X- und Y-Achse (33), Rotationsvorrichtung (31) und Halterung / Führung (32) für die Lichtwellenleiter (1). Sie dient der Bewegung des Lichtwellenleiters (1) auf die Fokussieroptik (24) zu bzw. von ihr weg. Auf diese Weise kann der Abstand des Fokuspunktes vom Mittelpunkt des Lichtwellenleiters (1), also die Tiefenposition, variiert werden. Die X-Achse (33) dient dem Verfahren des Lichtwellenleiters bzw. der Halterung / Führung (32) entlang des Lichtwellenleiterverlaufes unter der Fokussieroptik (24). Die maximale Länge eines modifizierten Bereiches wird also nur durch den maximalen Verfahrweg dieser Achse bestimmt. Die Y-Achse (33) bewegt die Halterung / Führung (32) rechtwinklig zum Lichtwellenleiterverlauf unter der Fokussieroptik (24). Sie dient der Kontrolle der Ausrichtung der Mikromodifikationen (5), da mit der Y-Achse (33) die Fokussieroptik (24) und der Lichtwellenleiter (1) so zueinander ausgerichtet werden können, dass der Laserstrahl (2) möglichst senkrecht auf die Lichtwellenleiteroberfläche trifft. Ein schräges Auftreffen auf die Oberfläche führt zu einem veränderten Strahlengang mit einer Verzerrung des Fokusbereiches und hat dadurch neben Einfluss auf die Ausrichtung auch Einfluss auf die Form und Größe der eingebrachten Modifikationen. Der verwendete Laserstrahl (20) wird meist über einen Umlenkspiegel (23) in die Fokussieroptik (24) geführt, was aber nicht zwingend ist. Der zu bearbeitende Lichtwellenleiter (1) wird über eine Halterung und Führung (32) in einer exakten Position vor der Fokussieroptik (24) gehalten. Diese Führung ist im Bereich der Bearbeitung ausgespart oder für die verwendete Laserstrahlung (2 bzw. 22) transparent. Die Rotationsvorrichtung (31) dient zur Rotation des Lichtwellenleiters (1) um seine Längsachse. Dazu wird der Lichtwellenleiter (1) mit einer Spannvorrichtung an der Rotationsvorrichtung (31) befestigt. Um eine übermäßige Torsionsspannung des Lichtwellenleiters (1) zu vermeiden, wird dieser dabei immer nur schrittweise um bis zu 360 Grad und anschließend um bis zu 360 Grad in Gegenrichtung gedreht. Dies ist sowohl für lose Lichtwellenleiterabschnitte, z.B. konfektionierte Lichtwellenleiter, als auch für Roll-to-Roll-Herstellungsprozesse realisierbar, in denen die Lichtwellenleiter (1) beliebig lang werden können.

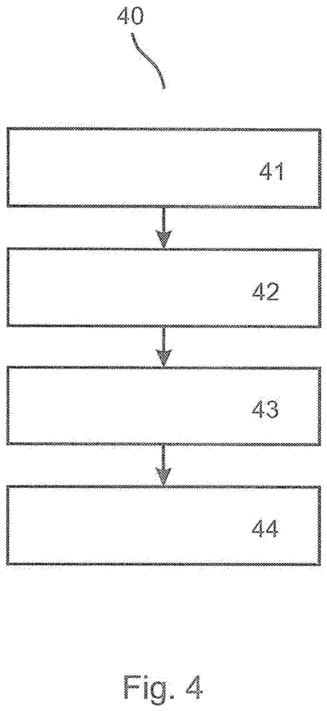

In Figur 4 ist in einer Ausführungsform der Erfindung ein Verfahren zur Bearbeitung von Lichtwellenleitern (1) mit Laserstrahlung (2) dargestellt. Zunächst wird der Lichtwellenleiter (1) mit Hilfe einer Halterung / Führung (32) in seiner Position fixiert (41). Die Halterung / Führung (32) ist so gestaltet, dass der Bereich des Lichtwellenleiters (1) der Laserstrahlung (2) zugänglich ist, in dem die Mikromodifikationen erzeugt werden sollen. Der Lichtwellenleiter wird so gelagert, dass er in drei Raumrichtungen gegenüber der Fokuslage bewegbar ist. Dies kann durch eine bewegliche Optik (24) und starre Lagerung des Lichtwellenleiters (1) erreicht werden, oder durch eine starre Optik (24) und einen beweglich angeordneten Lichtwellenleiter (1). Die Bewegungsmöglichkeiten umfassen die drei Raumrichtungen X, Y, und Z sowie die Rotation α um die Längsachse des Lichtwellenleiters (1) und/oder die Rotation β₁, β₂, β₃ um eine oder mehrere Achsen. In einem weiteren Verfahrensschritt (42) wird der Laserstrahl (2) fokussiert. Der fokussierte Laserstrahl (22) wird so positioniert, dass die Lage des Fokus mit Hilfe der Bewegungsmöglichkeiten durch den gesamten Bereich bewegbar ist, in dem Mikromodifikationen eingebracht werden sollen. Die Fokuslage wird durch den Lichtwellenleiter nach einem vorgegebenen Muster bewegt (43). Bevorzugt wird ein gepulster Laserstrahl verwendet. Durch eine kontinuierliche Bewegung der Fokuslage durch den Lichtwellenleiter (1) mit konstanter Geschwindigkeit entstehen Mikromodifikationen (5) mit in Bewegungsrichtung äquidistantem Abstand. Durch die Bewegung der Fokuslage durch den Lichtwellenleiter (1) nach einem vorgegebenen Muster werden 20 oder mehr Mikromodifikationen (5) erzeugt. In einem bevorzugten Ausführungsbeispiel der Erfindung werden durch die Bewegung der Fokuslage durch den Lichtwellenleiter (1) nach einem vorgegebenen Muster mehr als 36 Mikromodifikationen (5), besonders bevorzugt mehr als 360 Mikromodifikationen (5) erzeugt. In einem weiteren Verfahrensschritt die Bewegung der Fokuslage durch den Lichtwellenleiter (5) nach einem vorgegebenen Muster wiederholt (44).

In einer weiteren vorteilhaften Ausgestaltung wird nach Abschluss der durch die Bewegung der Fokuslage durch den Lichtwellenleiter (1) nach einem vorgegebenen Muster eingebrachten Mikromodifikationen (5) die Fokuslage gegenüber dem Lichtwellenleiter (1) durch eine Translations- und/oder Rotationsbewegung verändert.

Dies dient der Vermeidung, dass die Mikromodifikationen (5), die in dem Wiederholungsschritt durch die Bewegung der Fokuslage durch den Lichtwellenleiter (1) nach einem vorgegebenen Muster in den Lichtwellenleiter (1) eingebracht wurden, in Richtung der Lichtwellenleiterachse (17) genau hinter den Mikromodifikationen (5) liegen, die in dem ersten Schritt durch die Bewegung der Fokuslage durch den Lichtwellenleiter (1) nach einem vorgegebenen Muster in den Lichtwellenleiter (1) eingebracht wurden.

In einer weiteren vorteilhaften Ausgestaltung der Erfindung wird die kontinuierliche Bewegung der Fokuslage durch den Lichtwellenleiter (1) entlang der Lichtwellenleiterachse durchgeführt und ergibt so später eine der beschriebenen Anordnungen in der Schnittebene. Somit wird der Bearbeitungsvorgang innerhalb mehrerer Schnittebenen also in die Erzeugung einzelner Punkte bei jedem Durchgang entlang der Lichtwellenleiterachse (17) aufgeteilt.

In einer weiteren vorteilhaften Ausgestaltung der Erfindung wird die kontinuierliche Bewegung der Fokuslage durch den Lichtwellenleiter (1) nach einem vorgegebenen Muster durch eine weitere Bewegung überlagert. Diese Bewegung können beispielsweise Schwingungen sein, die dazu dienen, einen gewissen lateralen Offset zwischen den Mikromodifikationen (5) herzustellen, die in dem Wiederholungsschritt durch die Bewegung der Fokuslage durch den Lichtwellenleiter (1) nach einem vorgegebenen Muster in den Lichtwellenleiter (1) eingebracht wurden, und den Mikromodifikationen (5), die in dem ersten Schritt durch die Bewegung der Fokuslage durch den Lichtwellenleiter (1) nach einem vorgegebenen Muster in den Lichtwellenleiter (1) eingebracht wurden. Bevorzugt ist die Amplitude der Schwingung mindestens die Hälfte des Abstandes von benachbarten Mikromodifikationen (5). So entsteht eine geordnete Anordnung von Mikromodifikationen im Sinne der vorliegenden Erfindung,

Die Mikromodifikationen (5) werden so im Lichtwellenleiter (1) angeordnet, dass bei einer Durchstrahlung von Licht entlang der Lichtwellenleiterachse (17) durch den Lichtwellenleiter die Mikromodifikation derart angeordnet sind, dass das Licht durch die Mikromodifikationen möglichst vollständig zur Seite abgelenkt wird.

In einer weiteren vorteilhaften Ausführungsform der Erfindung werden die Mikromodifikationen (5) in den Lichtwellenleiter (1) eingebracht, indem die optische Achse (25) des Laserstrahls (2) bei der Bestrahlung des Lichtwellenleiters (1) den Lichtwellenleiter (1) abseits der Lichtwellenleiterachse (17) positioniert wird, Im Falle von Mikromodifikationen (5), deren Form deutlich von einer runden Form abweichen, also eher eine längliche Form aufweisen, kann so allein durch eine Rotationsbewegung eine nahezu geschlossene Fläche oder Linie von Mikromodifikationen (5) erreicht werden.

Bei einer weiteren vorteilhaften Ausgestaltung der Erfindung werden die Mikromodifikationen (5) in den Lichtwellenleiter (1) eingebracht, indem die optische Achse (25) des Laserstrahls (2) bei der Bestrahlung des Lichtwellenleiters (1) unter einem Winkel (β₁, β₂, β₃) auf den Lichtwellenleiter (1) treffen, der ungleich 90° ist. Bei Mikromodifikationen mit einer länglichen Form ergibt sich so ein spitzer Winkel zwischen der Orientierung der Mikromodifikation (5) und der Lichtwelienleiterachse (17). In einer weiteren Ausgestaltungsform der Erfindung liegt der Winkel (β₁, β₂, β₃) zwischen der Orientierung der Mikromodifikation (5) und der Lichtwellenleiterachse (17) in einem Bereich zwischen 10° und 80°, in einer bevorzugten Ausgestaltung in einem Bereich zwischen 20° und 70° und in einer besonders bevorzugten Ausgestaltungsform zwischen 30° und 60°.

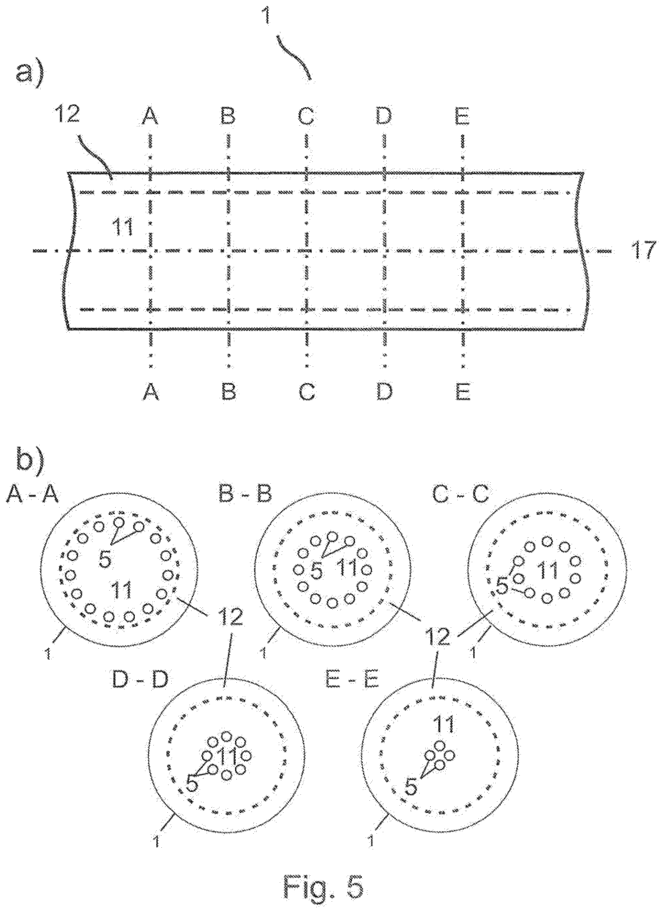

In Figur 5 ist der schematische Aufbau eines Lichtwellenleiters mit durch Laserstrahlung induzierten Mikromodifikationen (Teilbild a)) sowie Schnittbilder entlang den Schnittlinien A - A, B - B, C - C, D - D und E - E (Teilbild b)) dargestellt. Der Lichtwellenleiter (1) ist aufgebaut durch einen Kernbereich (11) und einen Mantelbereich (12). Durch die Bestrahlung gemäß des erfindungsgemäßen Verfahrens (40) wurden Mikromodifikationen (5) in den Kernbereich (12) des Lichtwellenleiters (1) eingebracht. Die Mikromodifikationen (5) auf den dargestellten Schnittebenen (A - A, B - B, C - C, D - D und E - E) sind rotationssymmetrisch um die Lichtwellenleiterachse (17) angeordnet. Die Mikromodifikationen (5) haben auf jeder Schnittebene den gleichen Abstand zur Lichtwellenleiterachse (17) und sind auf einem Kreisbogen um die Lichtwellenleiterachse (17) angeordnet. In der Schnittebene A - A liegen die Mikromodifikationen (5) nahe zum Mantel (12) des Lichtwellenleiters (1) und haben einen großen Abstand zur Lichtwellenleiterachse (17). Im Verlauf über die Schnittebenen B - B bis E - E vergrößert sich der Abstand der Mikromodifikationen (5) zum Mantel (12) des Lichtwellenleiters (1) bzw. verringert sich der Abstand der Mikromodifikationen (5) zur Lichtwellenleiterachse (17). In einer weiteren vorteilhaften Ausgestaltung der Erfindung verringert sich die Anzahl der auf einem Kreisbogen in einer Schnittebene angeordneten Mikromodifikationen (5) mit dem Abstand der Mikromodifikationen (5) zur Lichtwellenleiterachse (17). Dies wird dadurch erreicht, dass das Zeitintervall zwischen zwei Laserpulsen verändert wird und/oder die Rotationsgeschwindigkeit verändert wird.

In einer weiteren vorteilhaften Ausgestaltung der Erfindung erfolgt die Anordnung der Mikromodifikationen in nur einer der hier dargestellten Schnittebenen (z.B. A - A) entlang des gesamten Lichtwellenleiters bzw. in mehreren Kreisen ineinander, d.h. als in einer Schnittebene kombinierte Anordnungen von hier dargestellten Schnittebenen (z.B. A A mit C C und/oder E E),

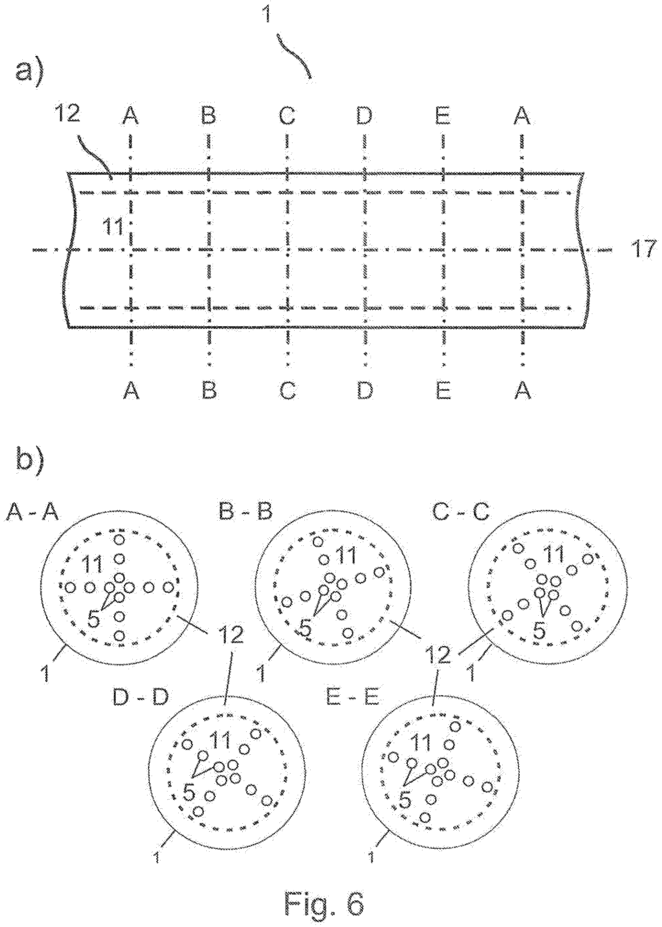

Figur 6 zeigt in Teilbild a) den schematischen Aufbau eines Lichtwellenleiters mit durch Laserstrahlung induzierten Mikromodifikationen, In Teilbild b) sind die Querschnitte entlang den Schnittlinien A - A, B - B, C - C, D - D und E - E dargestellt. Der Lichtwellenleiter (1) ist aufgebaut durch einen Kernbereich (11) und einen Mantelbereich (12). Durch die Bestrahlung mit hochenergetischer Strahlung wurden gemäß des erfindungsgemäßen Verfahrens (40) Mikromodifikationen (5) in den Kernbereich (12) des Lichtwellenleiters (1) eingebracht. Die Mikromodifikationen (5) auf den dargestellten Schnittebenen (A - A, B - B, C - C, D - D und E - E) sind rotationssymmetrisch um die Lichtwellenleiterachse (17) angeordnet. Anzahl und Anordnung der Mikromodifikationen (5) sind in jeder Schnittebene gleich. Die Anordnung der Mikromodifikationen (5) auf Schnittebene B - B ist gegenüber der Anordnung der Mikromodifikationen (5) auf Schnittebene A - A um einen Winkel um die Lichtwellenleiterachse (17) verdreht. Diese Rotation der Anordnungen der Mikromodifikationen (5) kann durch eine Rotation des Lichtwellenleiters zwischen den Bearbeitungsintervallen zur Einbringung der Mikromodifikationen (5) in den Lichtwellenleiter (1) erreicht werden. Im Verlauf über die Schnittebenen A - A bis E - E vergrößert sich der Rotationswinkel der einzelnen Schnittebenen B - B bis E - E gegenüber Schnittebene A - A. In einer weiteren vorteilhaften Ausgestaltung der Erfindung ist die Anzahl der Schnittebenen A - A bis E - E mit unterschiedlichen Rotationswinkeln in einem Bearbeitungsintervall so gewählt, dass die Anordnung der Mikromodifikationen (5) der letzten Schnittebene des Bearbeitungsintervalls E - E bei Fortführung der Rotation wieder zur Anordnung der Mikromodifikationen (5) auf der ersten Schnittebene A - A des Bearbeitungsintervalls führen würde.

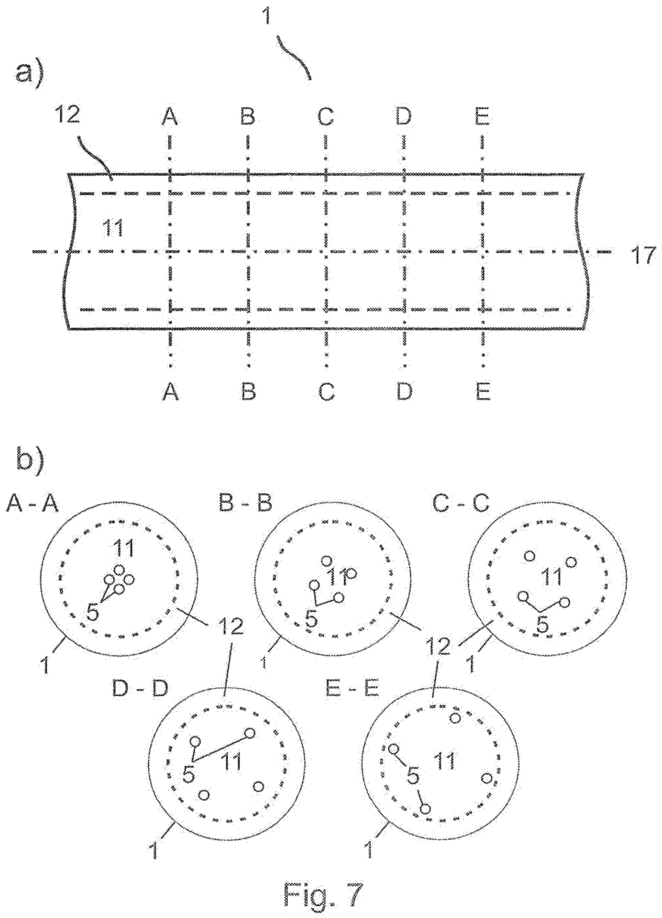

In Figur 7 ist der schematische Aufbau eines Lichtwellenleiters mit durch Laserstrahlung induzierten Mikromodifikationen (Teilbild a)) sowie Schnittbilder entlang den Schnittlinien A - A, B - B, C - C, D - D und E - E (Teilbild b)) dargestellt. Der Lichtwellenleiter (1) ist aufgebaut durch einen Kernbereich (11) und einen Mantelbereich (12). Durch die Bestrahlung gemäß des erfindungsgemäßen Verfahrens (40) wurden Mikromodifikationen (5) in den Kernbereich (12) des Lichtwellenleiters (1) eingebracht. Die Mikromodifikationen (5) auf den einzelnen Schnittebenen (A - A, B B, C - C, D - D und E - E) sind rotationssymmetrisch um die Lichtwellenleiterachse (17) angeordnet. Die Mikromodifikationen (5) sind auf den einzelnen Schnittebenen A A bis E - E auf Kreisbögen um die Lichtwellenleiterachse (17) angeordnet. Die Radien der Kreisbögen verändern sich im Verlauf der Schnittebenen A - A bis E - E. Weiterhin ist die Anordnung der Mikromodifikationen (5) auf einer Schnittebene B - B gegenüber der Anordnung der Mikromodifikationen (5) auf einer benachbarten Schnittebene A - A um einen Winkel um die Lichtwellenleiterachse (17) verdreht. Zur Überführung der Bearbeitungsschritte zur Anordnung der Mikromodifikationen (5) auf einer Schnittebene A - A zu den Bearbeitungsschritten zur Anordnung der Mikromodifikationen (5) auf einer benachbarten Schnittebene B - B ist eine Kombination aus einer Rotation um die Lichtwellenleiterachse (17) und Translation des fokussierten Laserstrahls (22) gegenüber dem Lichtwellenleiter (1) zwischen den Bearbeitungsschritten zur Anordnung der Mikromodifikationen (5) auf den benachbarten Schnittebenen möglich.

In einer weiteren vorteilhaften Ausgestaltung der Erfindung erfolgt die Anordnung der Mikromodifikationen in nur einer der hier dargestellten Schnittebenen (z.B. A - A) entlang des gesamten Lichtwellenleiters jedoch gedreht um die Lichtwellenleiterachse bzw. von einer Kombination von Schnittebenen, d.h. als in einer Schnittebene kombinierte Anordnungen von hier dargestellten Schnittebenen (z.B. A A mit C C und/oder E E). In einer weiteren vorteilhaften Ausgestaltung der Erfindung erfolgt die Anordnung der Mikromodifikationen erfolgt die Anordnung in einer Kombination von Schnittebenen, d.h. als in einer Schnittebene kombinierte Anordnungen von hier dargestellten Schnittebenen (z.B. A A mit C C und/oder E E), die jedoch mit jeder weiteren Schnittebene nach dem beschriebenen Muster der einzelnen Schnittebenen wechselt.

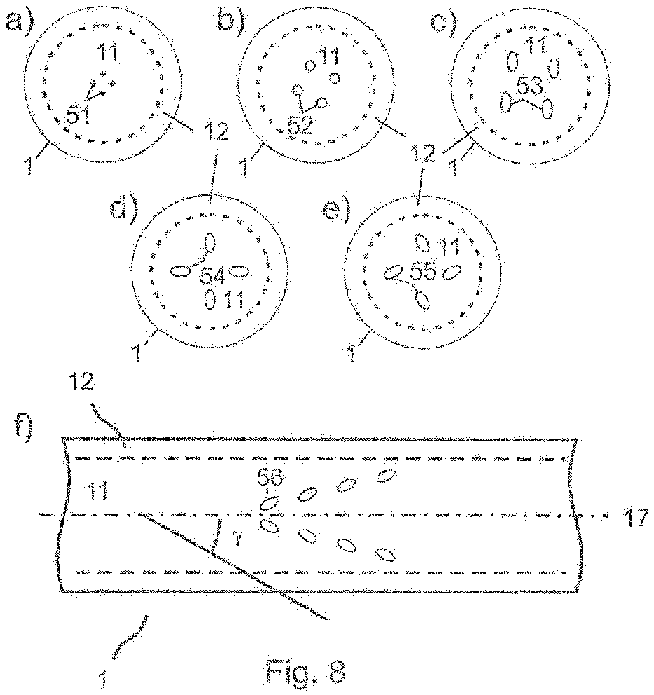

Figur 8 zeigt anhand von Querschnittsbildern mit Schnitten senkrecht zur Lichtwellenleiterachse (17) (Teilbilder a) bis e)) und einem Längsschnitt entlang der Lichtwellenleiterachse (17) (Teilbild f)) jeweils verschiedene Ausführungsformen der Erfindung, in denen unterschiedliche Ausgestaltungen von Mikromodifikationen (5) dargestellt sind, die durch Laserstrahlung in einen Lichtwellenleiter induziert wurden. Die Teilbilder a) und b) zeigen Mikromodifikationen (51, 52) mit unterschiedlichen Größen. Die Lage der Mikromodifikationen kann unabhängig davon gewählt werden. Die Größe der Mikromodifikationen (51, 52) können durch die Größe des Fokus und/oder durch die Menge an eingebrachter Energie beeinflusst werden. Die Energie für den Einzelpuls kann zwischen 1 und 50 µJ betragen und die Mikromodifikationen werden mit zunehmender Energie größer, was aber abhängig von Material des Lichtwellenleiters und der Laserstrahlgüte ist. Weiterhin besteht die Möglichkeit Mikromodifikationen (5) so anzuordnen, dass sich ihre Grenzflächen berühren oder überlappen. Durch die Form und Positionierung des Fokus kann auch die Form der Mikromodifikation (52, 53) beeinflusst werden. Bei einem sehr langegezogenen Fokus entstehen Mikromodifikationen (53), die einen ellipsoiden Querschnitt mit einem hohen Verhältnis von Länge zu Breite aufweisen, während bei einer kurzen Fokuslänge Mikromodifikationen (52) entstehen die ein kleines Verhältnis von Länge zu Breite aufweisen. Mit der Form der Mikromodifikationen (53, 54, 55, 56) besteht ein weiterer Parameter, der für die Herstellung einer geordneten Anordnung der Mikromodifikationen (5) herangezogen werden kann. In den Teilbildern c) bis f) sind unterschiedliche Orientierungen der Längsrichtung der Mikromodifikationen (53, 54, 55, 56) dargestellt. In Teilbild c) sind die Mikromodifikationen (53) alle in die gleiche Richtung orientiert. Dies wird erreicht, wenn zwischen den Pulsen der Laserstrahlung eine in Y-Richtung laterale Translation zwischen Lichtwellenleiter (1) und Fokuslage durchgeführt wird und die infolge der durch den schräg auf die Oberfläche des Lichtwellenleiters (1) auftreffenden fokussierten Laserstrahl (22) entstehenden Brechung durch eine geeignete Rotation von dem fokussierten Laserstrahl (22) um β₁, β₂, β₃ ausgeglichen wird. In Teilbild d) sind die Orientierungen der Mikromodifikationen (54) rotationssymmetrisch um die Lichtwellenleiterachse (17) des Lichtwellenleiters (1) angeordnet. In der Bearbeitung wird dies erreicht, indem zwischen den Laserpulsen der Lichtwellenleiter (1) um die Lichtwellenleiterachse (17) rotiert. Die Mikromodifikationen (54) sind so orientiert, dass die Achse entlang der Längsrichtung der Mikromodifikation (54) durch das Zentrum der Mikromodifikation (54) die Lichtwellenleiterachse (17) des Lichtwellenleiters (1) schneidet. In Teilbild e) ist die Anordnung und Orientierung der Mikromodifikationen (5) gezeigt, wenn zusätzlich zu dem Bearbeitungsverfahren für Teilbild d) die fokussierte Laserstrahlung (22) nicht in Richtung der Lichtwellenleiterachse (17) eingebracht wird, sondern der Lichtwellenleiter (1) lateral zur Lichtwellenleiterachse (17) verschoben ist. Die Mikromodifikationen (55) sind dann so orientiert, dass eine Achse entlang der Längsrichtung der Mikromodifikation (55) nicht die Lichtwellenleiterachse (17) des Lichtwellenleiters (1) schneidet. Teilbild f) zeigt Mikromodifikationen (56), deren Achse entlang der Längsrichtung der Mikromodifikation (56) durch das Zentrum der Mikromodifikation (56) zur Lichtwellenleiterachse (17) einen spitzen Winkel (γ) bildet. Der Winkel (γ) zwischen der Orientierung der Mikromodifikation (5) und der Lichtwellenleiterachse (17) liegt in einem Bereich zwischen 10° und 80°, in einer bevorzugten Ausgestaltung in einem Bereich zwischen 20° und 70° und in einem besonders bevorzugten Ausgestaltungsform zwischen 30° und 60°, Die Ausrichtung der Winkel (γ) kann mit der Spitze zum distalen oder proximalen Ende des Lichtwellenleiters (1) erfolgen. Die Anordnung der Mikromodifikationen kann rotationssymmetrisch zur Lichtwellenleiterachse (17) ausgestaltet sein und zum distalen und proximalen Ende des Lichtwellenleiters (1) enger werden.

Ein Bewegungsmuster zur Anordnung und/oder Orientierung von Mikromodifikationen (5, 51, 52, 53, 54, 55, 56) in einem Lichtwellenleiter (1) weist eine oder mehrere Bewegungen aus der Gruppe umfassend eine Translation entlang der Raumrichtungen X, Y und/oder Z und/oder Rotationen um die Lichtwellenleiterachse (17) und/oder eine Achse senkrecht zur Lichtwellenleiterachse (17) auf. Innerhalb eines Bewegungsmusters wird mindestens eine Mikromodifikation (5, 51, 52, 53, 54, 55, 56) im Kern (11) des Lichtwellenleiters (1) erzeugt. Zwischen der ersten Ausführung des Bewegungsmusters und der zweiten und/oder einer nachfolgenden Wiederholung der Ausführung des Bewegungsmusters erfolgt eine oder mehrere Bewegungen aus der Gruppe umfassend eine Translation entlang der Raumrichtungen X, Y und/oder Z und/oder Rotationen um die Lichtwellenleiterachse (17) und/oder eine Raumachse. Dabei kann sich der Bereich, in dem Mikromodifikationen (5, 51, 52, 53, 54, 55, 56) in einem ersten Bewegungsmuster in den Lichtwellenleiter (1) eingebracht wurden, und der Bereich, in dem Mikromodifikationen (5, 51, 52, 53, 54, 55, 56) in einem zweiten Bewegungsmuster in den Lichtwellenleiter (1) eingebracht wurden, überschneiden.

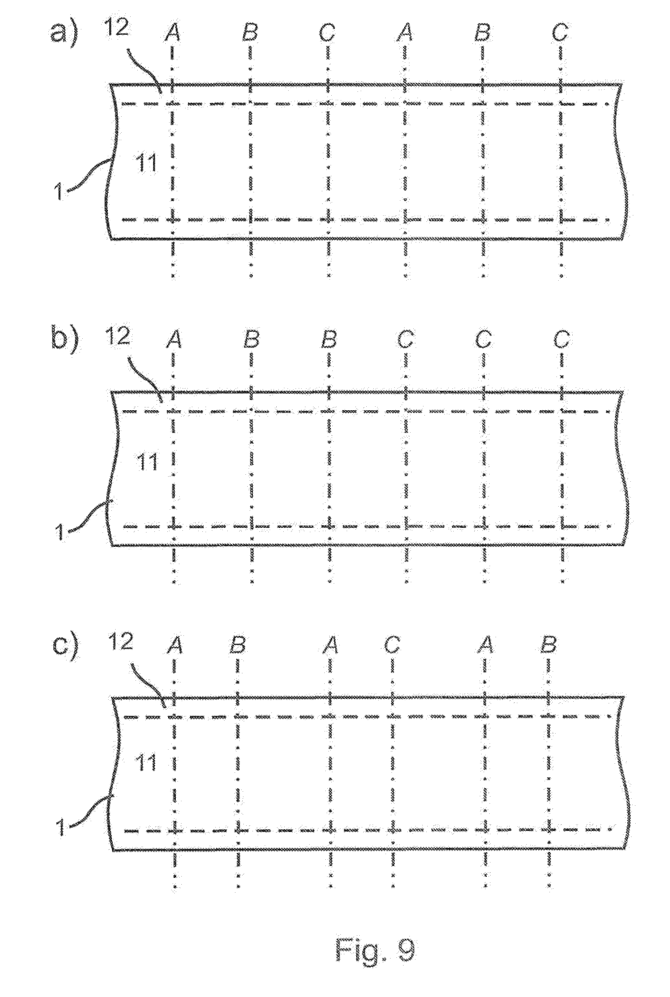

Figur 9 zeigt in Teilbildern a) bis c) den schematischer Aufbau eines Lichtwellenleiters (1) mit durch Laserstrahlung induzierten Mikromodifikationen (5). Die gekennzeichneten Schnittlinien *A, B und C* stehen kennzeichnend für Bereiche, in die in Folge eines Bewegungsmusters der Fokuslage des fokussierten Laserstrahls (22) durch den Lichtwellenleiter (1) Mikromodifikationen (5) in den Lichtwellenleiter (1) eingebracht wurden. In Teilbild a) ist eine Abfolge von exemplarisch drei unterschiedlichen Bereichen (*A, B, C*) von Anordnungen von Mikromodifikationen (5) dargestellt, die sich über die Länge des Lichtwellenleiters noch einmal wiederholen. Die Anzahl der Wiederholungen kann auch mehrfach erfolgen. Die Bereiche (*A*, *B, C*) weisen unterschiedliche Anordnungen der Mikromodifikationen auf. Dabei wird ein Bereich (A, B, C) durch einen oder mehrere der Charakteristika aus der Gruppe umfassend die Größe, Anzahl, Orientierung, Form und/oder der Anordnung der Mikromodifikationen (5, 51, 52, 53, 54, 55, 56) definiert. Die Mikromodifikationen (5, 51, 52, 53, 54, 55, 56) eines jeden Bereiches *(A, B, C*) werden durch ein Bewegungsmuster der Fokuslage der fokussierten Laserstrahlung (22) durch den Lichtwellenleiter (1) und der damit verbunden Bestrahlung induziert. Infolge der unterschiedlichen Anordnungen der Mikromodifikationen (5, 51, 52, 53, 54, 55, 56) werden die Anordnungen der Mikromodifikationen (5, 51, 52, 53, 54, 55, 56) in den Bereichen (*A, B, C*) durch unterschiedliche Bewegungsmuster erstellt. Zwischen der ersten Ausführung eines Bewegungsmusters zur Herstellung eines Bereichs (*A, B, C*) erfolgt eine oder mehrere von Bewegungen der Fokuslage in Bezug auf den Lichtwellenleiter (1) aus einer Gruppe umfassend die drei Raumrichtungen X, Y, und Z sowie die Rotation α um die Längsachse des Lichtwellenleiters (1) und die Rotation β₁, β₂, β₃ um eine oder mehrere Achsen.

In Teilbild b) von Figur 9 ist in einer weiteren Ausführungsform der Erfindung eine weitere Abfolge von Bereichen (*A*, *B, C*) gleicher Anordnung von Mikromodifikationen (5, 51, 52, 53, 54, 55, 56) in einem Lichtwellenleiter (1) dargestellt. Während der erste Bereich (*A*) einmal vorliegt, folgen zwei Bereiche mit einer zweiten Anordnung (*B*) und drei Bereiche mit einer dritten Anordnung (*C*). In diesem bearbeiteten Lichtwellenleiter (1) sind nicht alle Bereiche mit nach einem bestimmten Muster angeordneten Mikromodifikationen (5, 51, 52, 53, 54, 55, 56) mehrfach vorhanden.

Teilbild c) der Figur 9 zeigt in einer weiteren Ausgestaltung der Erfindung eine andere mögliche Abfolge von Bereichen (*A, B, C*) gleicher Anordnung von Mikromodifikationen (5, 51, 52, 53, 54, 55, 56). Während der erste Bereich (*A*) auf jeden Bereich (*A, B, C*) folgt, der nicht gleich dem ersten Bereich (*A*) ist, folgen die zweiten und dritten Bereiche (*B, C*) im Wechsel auf den ersten Bereich (*A*)*.*

Weitere Ausführungsformen der Erfindung lassen sich durch beliebige mathematische Reihen und Folgen darstellen. Dabei umfasst ein erfindungsgemäßer Lichtwellenleiter (1) in einer weiteren Ausgestaltung der Erfindung mehr als drei Bereiche (*A, B, C*) mit unterschiedlichen Anordnungen von Mikromodifikationen (5, 51, 52, 53, 54, 55, 56). In einer bevorzugten Ausführungsform der Erfindung umfasst der Lichtwellenleiter (1) mehr als fünf Bereiche (*A, B, C*), in einer besonders bevorzugten Ausführungsform mehr als zehn Bereiche (*A, B, C*) mit unterschiedlich angeordneten Mikromodifikationen (5, 51, 52, 53, 54, 55, 56).

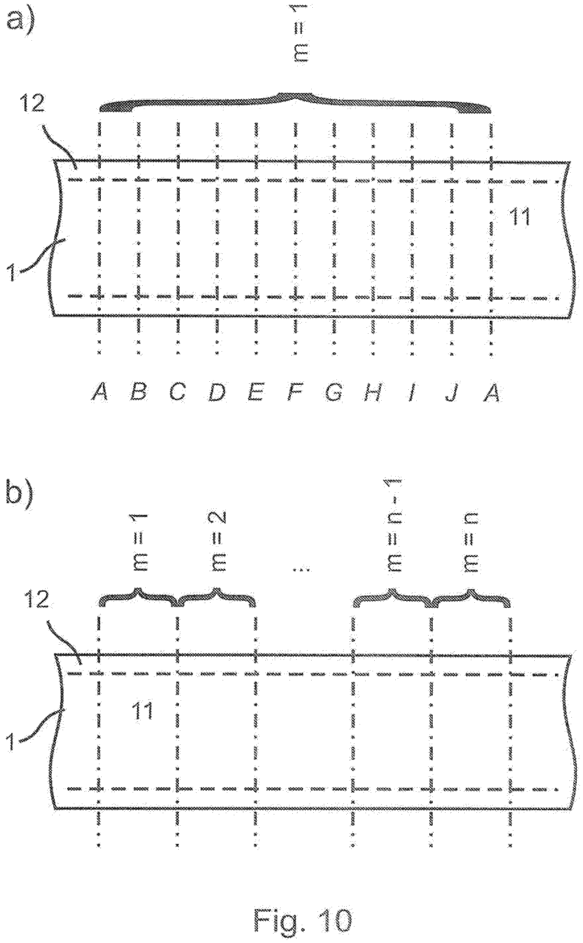

Figur 10 zeigt in den Teilbildern a) und b) den schematischen Aufbau eines Lichtwellenleiters (1) mit durch fokussierte Laserstrahlung (22) induzierten Mikromodifikationen (5, 51, 52, 53, 54, 55, 56). In Teilbild a) ist eine Abfolge mit einer Vielzahl von Bereichen (*A, B, C, D, E, F, G, H, I, J*) mit unterschiedlicher Anordnung von Mikromodifikationen (5, 51, 52, 53, 54, 55, 56) dargestellt. Diese Abfolge von Bereichen (*A, B, C, D, E, F,* G, *H, I, J*) mit unterschiedlicher Anordnung von Mikromodifikationen (5, 51, 52, 53, 54, 55, 56) wird n-mal wiederholt (Teilbild b)). n, m sind hierbei natürliche Zahlen. m steht für die Anzahl der Wiederholungen der eine Abfolge mit einer Vielzahl von Bereichen (*A, B, C, D, E, F, G H, I, J*) mit unterschiedlicher Anordnung von Mikromodifikationen (5, 51, 52, 53, 54, 55, 56).

In einer bevorzugten Ausführungsform der Erfindung ist die Anzahl der Wiederholungen einer Abfolge mit einer Vielzahl von Bereichen (*A, B, C, D, E, F, G, H, I, J*) mit unterschiedlicher Anordnung von Mikromodifikationen (5, 51, 52, 53, 54, 55, 56) größer als fünf, in einer besonders bevorzugten Ausführungsform größer als zwanzig.

In einer weiteren Ausführungsform der Erfindung ist die Anordnung der Wiederholungen einer Abfolge mit einer Vielzahl von Bereichen (*A, B, C, D, E, F*, *G, H, I, J*) mit unterschiedlicher Anordnung von Mikromodifikationen (5, 51, 52, 53, 54, 55, 56) alternierend in der Ausrichtung ihrer Anordnung.

In einer weiteren Ausführungsform der Erfindung ist die Anordnung der Wiederholungen einer Abfolge mit einer Vielzahl von Bereichen (*A, B, C, D, E, F, G, H, I, J*) mit unterschiedlicher Anordnung von Mikromodifikationen (5, 51, 52, 53, 54, 55, 56) eine Mischform von alternierender und gleichsinniger Ausrichtung ihrer Anordnung.

### BEZUGSZEICHENLISTE

- 1: Lichtwellenleiter
- 11: Kern des Lichtwellenleiters
- 12: Mantel des Lichtwellenleiters
- 13: Coating, Buffer und/oder weitere Beschichtungen des Lichtwellenleiters
- 14: Endkappe
- 15: Proximales Ende des Lichtwellenleiters
- 16: Distales Ende des Lichtwellenleiters
- 17: Lichtwellenleiterachse
- 2: Laserstrahl
- 20: Vorrichtung zum Einbringen von Mikromodifikationen in Lichtwellenleiter
- 21: symbolisierte Fokussieroptik
- 22: Fokussierter Laserstrahl
- 23: Umlenkspiegel
- 24: Fokussieroptik
- 25: Optische Achse
- 31: Rotationsvorrichtung
- 32: Halterung / Führung für den Lichtwellenleiter
- 33: Laterale Positioniervorrichtung
- 34: Vertikale Positioniervorrichtung
- α: Rotation des Lichtwellenleiters um die Lichtwellenleiterachse
- β₁, β₂, β₃: Rotation der Einstrahlrichtung des Laserstrahls
- 40: Verfahren zur Einbringung von Mikromodifikationen in Lichtwellenleitern
- 41: Fixieren des Lichtwellenleiters in einer Halterung
- 42: Fokussieren von Laserstrahlung in einer Fokuslage
- 43: Bewegen der Fokuslage durch den Lichtwellenleiter nach einem vorgegebenen Muster
- 44: Wiederholung einer der Bewegungen der Fokuslage durch den Lichtwellenleiter nach einem vorgegebenen Muster
- 5, 51, 52, 53,: Mikromodifikation
- 54, 55, 56, γ: Winkel der Längsausrichtung der Mikromodifikationen zur Lichtwellenleiterachse
- A, B, C, D, E: Radiale Schnittebenen durch den Lichtwellenleiter, können auch geneigt sein
- F: Axiale Schnittebene durch den Lichtwellenleiter
- *A, B, C, D,*: Bereiche des Lichtwellenleiter mit darin angeordneten
- *E, F, G, H, J*: Mikromodifikationen
- m: Anzahl der Wiederholungen einer Abfolge mit einer Vielzahl von Bereichen mit unterschiedlicher Anordnung von Mikromodifikationen
- n: maximale Anzahl der Wiederholungen einer Abfolge mit einer Vielzahl von Bereichen mit unterschiedlicher Anordnung von Mikromodifikationen

Beispiel 1: Lichtwellenleiter umfassend
einen lichtwellenführenden Kern,
einen Bereich im Lichtwellenleiter,
wobei in dem Bereich des Lichtwellenleiters Mikromodifikationen angeordnet sind,
wobei die Anordnung der Mikromodifikationen geordnet ist.

Beispiel 2: Lichtwellenleiter nach Beispiel 1,
dadurch gekennzeichnet, dass
die Mikromodifikationen auf einer oder mehreren Schnittebenen angeordnet sind, wobei die Schnittebenen im Wesentlichen senkrecht zur Lichtwellenleiterachse liegen und,
wobei die Anordnung der Mikromodifikationen auf der Schnittebene durch einen oder mehrere Parameter aus einer Gruppe von Parametern umfassend die symmetrische Anordnung der Mikromodifikationen, die Dichte der
Mikromodifikationen auf der Schnittebene, die Größe der Mikromodifikationen, den Abstand der Mikromodifikationen zur Lichtwellenleiterachse, den Abstand der Mikromodifikationen zueinander, der Ausrichtung der Mikromodifikationen oder anderer Parameter, mit deren Hilfe die Lage und Verteilung der Mikromodifikationen oder deren Größe oder äußere Form beschrieben wird.

Beispiel 3: Lichtwellenleiter nach Beispiel 1 oder 2
dadurch gekennzeichnet, dass
die Anordnung der Mikromodifikationen auf einer ersten Schnittebene auf mindestens einer anderen Schnittebene wiederholt wird.

Beispiel 4: Lichtwellenleiter nach Beispiel 3 dadurch gekennzeichnet, dass
die Schnittebene, auf der die Anordnung der Mikromodifikationen auf der ersten Schnittebene wiederholt wird, gegenüber der ersten Schnittebene um einen Winkel verdreht ist.

Beispiel 5: Lichtwellenleiter nach Beispiel 3
dadurch gekennzeichnet, dass
der Abstand zwischen der ersten Schnittebene und der anderen Schnittebene, auf der die Anordnung der Mikromodifikationen wiederholt wird, größer ist als die Ausdehnung einer Mikromodifikation.

Beispiel 6: Lichtwellenleiter nach einem der Bespiele 3 oder 4
dadurch gekennzeichnet, dass
zwischen der ersten Schnittebene und der Schnittebene, auf der die Anordnung der Mikromodifikationen der ersten Schnittebene wiederholt wird, mindestens eine weitere Schnittebene mit Mikromodifikationen liegt, die eine andere Anordnung als die erste Schnittebene aufweist.

Beispiel 7: Lichtwellenleiter nach einem oder mehreren der Beispiele 2 bis 6 dadurch gekennzeichnet, dass
die Mikromodifikationen auf der Schnittebene rotationssymmetrisch um die Lichtwellenleiterachse angeordnet sind.

Beispiel 8: Lichtwellenleiter nach einem oder mehreren der Beispiele 2 bis 6 dadurch gekennzeichnet, dass
die Mikromodifikationen auf einen Hohlkegel angeordnet sind, wobei die Längsachse des Hohlkegels auf der Lichtwellenleiterachse liegt.

Beispiel 9: Lichtwellenleiter nach einem oder mehreren der Beispiele 2 bis 6 dadurch gekennzeichnet, dass die Mikromodifikationen auf mehreren Hohlkegeln angeordnet sind,
wobei die Hohlkegel unterschiedliche Durchmesser aufweisen und,
wobei die Längsachsen der Hohlkegel auf der Lichtwellenleiterachse liegen.

Beispiel 10: Lichtwellenleiter nach einem oder mehreren der vorangehenden Beispiele
dadurch gekennzeichnet, dass
der Bereich im Lichtwellenleiter, in dem Mikromodifikationen angeordnet sind, in Richtung der Lichtwellenleiterachse in mindestens zwei Abschnitte unterteilt ist,

in denen verschiedene Ausrichtungen und Ausführungsformen geordneter Mikromodifikationen eingebracht sind.

Beispiel 11: Verfahren zur Einbringung von Mikromodifikationen in Lichtwellenleitern umfassend
Fixierung eines Lichtwellenleiters in einer Halterung, wobei der Lichtwellenleiter und/oder die Halterung beweglich gelagert sind,
Fokussieren von hochenergetischer Strahlung in eine Fokuslage, wobei die Fokuslage im Inneren des Lichtwellenleiters positionierbar ist, wobei die Strahlung von einer Strahlungsquelle im Pulsbetrieb erzeugt wird,
wobei die Fokussiereinrichtung zum Fokussieren der hochenergetischen Strahlung beweglich gelagert ist
Bewegen der Fokuslage durch den Lichtwellenleiter dadurch gekennzeichnet, dass
die Bewegung der Fokuslage im Inneren des Lichtwellenleiters in Abhängigkeit der Repetitionsrate gewählt wird.

Beispiel 12: Verfahren zur Einbringung von Mikromodifikationen in Lichtwellenleitern nach Beispiel 11
dadurch gekennzeichnet, dass die Bewegung des Lichtwellenleiters in einer Rotationsbewegung ausgeführt wird.

Beispiel 13: Verfahren zur Einbringung von Mikromodifikationen in Lichtwellenleitern nach Beispiel 11 oder 12
dadurch gekennzeichnet, dass
die Fokuslage kontinuierlich durch den Lichtwellenleiter bewegt wird.

Beispiel 14: Verfahren zur Einbringung von Mikromodifikationen in Lichtwellenleitern nach einem oder mehreren der Beispiele 11 bis 13
dadurch gekennzeichnet, dass
die Bewegung der Fokuslage durch den Lichtwellenleiter eine Kombination aus Rotations- und Translationsbewegung ist.

Beispiel 15: Verfahren zur Einbringung von Mikromodifikationen in Lichtwellenleitern nach einem oder mehreren der Beispiele 11 bis 14
dadurch gekennzeichnet, dass
die Positionierung der Fokuslage in dem Lichtwellenleiter so mit der
Repetitionsrate korreliert, dass eine geordnete Anordnung von
Mikromodifikationen im Lichtwellenleiter entsteht.

Beispiel 16: Verfahren zur Einbringung von Mikromodifikationen in Lichtwellenleitern nach Beispiel 15
dadurch gekennzeichnet, dass
die Anordnung der Mikromodifikationen auf der Schnittebene durch einen oder mehrere Parameter aus einer Gruppe von Parametern umfassend die symmetrische Anordnung der Mikromodifikationen, die Dichte der
Mikromodifikationen auf der Schnittebene, die Größe der Mikromodifikationen, den Abstand der Mikromodifikationen zur Lichtwellenleiterachse, den Abstand der Mikromodifikationen zueinander, der Ausrichtung der Mikromodifikationen oder anderer Parameter, mit deren Hilfe die Lage und Verteilung der
Mikromodifikationen oder deren Größe oder äußere Form beschrieben wird.

Beispiel 17: Verfahren zur Einbringung von Mikromodifikationen in Lichtwellenleitern nach einem oder mehreren der Beispiele 11 bis 16
dadurch gekennzeichnet, dass
die Einstrahlrichtung der Strahlung auf den Lichtwellenleiter unter einen Winkel zwischen Lichtwellenleiterachse und Einstrahlrichtung von ungleich 90° erfolgt, in einem bevorzugten Bereich unter einem Winkel von ungleich 90° +/- 5° erfolgt, in einem besonders bevorzugten Bereich unter einem Winkel von ungleich 90° +/- 10° erfolgt.

Beispiel 18: Verfahren zur Einbringung von Mikromodifikationen in Lichtwellenleitern nach einem oder mehreren der Beispiele 11 bis 17
dadurch gekennzeichnet, dass
die Fokussiereinrichtung zusätzlich in lateraler und transversaler Richtung in Schwingungen versetzt wird.

## Patentansprüche

1. Lichtwellenleiter (1) umfassend
- einen lichtwellenführenden Kern (11),
- einen Bereich (15) im Lichtwellenleiter (1),
wobei in dem Bereich des Lichtwellenleiters (1) Mikromodifikationen (5, 51, 52, 53, 54, 55, 56) angeordnet sind.

2. Lichtwellenleiter nach Anspruch 1, wobei die Anordnung der Mikromodifikationen (5) geordnet ist.

3. Lichtwellenleiter nach einem oder mehreren der Ansprüche 1 bis 2, wobei die Mikromodifikationen (5) eine unregelmäßige Verteilung der Mikromodifikationen (5) umfasst.

4. Lichtwellenleiter nach einem oder mehreren der Ansprüche 1 bis 3, wobei Schnittebenen (A-A, B-B, C-C, D-D, E-E) im Wesentlichen senkrecht zu einer Lichtwellenleiterachse (17) liegen und die Mikromodifikationen auf jeder der Schnittebenen (A-A, B-B, C-C, D-D, E-E) so angeordnet sind, dass die Mikromodifikationen mindestens zwei unterschiedliche Abstände von der Lichtwellenleiterachse haben.

5. Lichtwellenleiter nach Anspruch 4, wobei die Schnittebenen so angeordnet sind, dass die Mikromodifikationen spiralförmig entlang der Lichtwellenleiterachse (17) angeordnet sind.

6. Lichtwellenleiter nach einem oder mehreren der Ansprüche 4 bis 5, wobei ein Abstand zwischen den Mikromodifikationen auf mindestens einer Schnittebene im Vergleich zu einer nachfolgenden Schnittebene variiert wird.

7. Lichtwellenleiter nach einem oder mehreren der Ansprüche 1 bis 6, wobei die Anordnung der Mikromodifikationen auf einer ersten Schnittebene auf mindestens einer anderen Schnittebene wiederholt wird, und/oder oder eine Anzahl und Anordnung der Mikromodifikationen (5) in jeder Schnittebene gleich sind.

8. Lichtwellenleiter nach Anspruch 7, wobei die Schnittebene, auf der die Anordnung der Mikromodifikationen auf der ersten Schnittebene wiederholt wird, gegenüber der ersten Schnittebene um einen Winkel verdreht ist.

9. Lichtwellenleiter nach Anspruch 7, wobei der Abstand zwischen der ersten Schnittebene und der anderen Schnittebene, auf der die Anordnung der Mikromodifikationen wiederholt wird, größer ist als die Ausdehnung einer Mikromodifikation.

10. Lichtwellenleiter nach einem der Ansprüche 7 bis 9, wobei
zwischen der ersten Schnittebene und der Schnittebene, auf der die Anordnung der Mikromodifikationen der ersten Schnittebene wiederholt wird, mindestens eine weitere Schnittebene mit Mikromodifikationen liegt, die eine andere Anordnung als die erste Schnittebene aufweist; oder
eine Abfolge von m Bereichen (A, *B,* C, D, *E, F, G, H, I, J*) mit unterschiedlicher Anordnung von Mikromodifikationen (5, 51, 52, 53, 54, 55, 56) n-mal wiederholt wird, wobei n und m natürliche Zahlen sind.

11. Verfahren zur Einbringung von Mikromodifikationen (5, 51, 52, 53, 54, 55, 56) in einen Lichtwellenleiter (1), wobei das Verfahren umfasst:
Fixieren des Lichtwellenleiters (1) in einer Halterung (32), wobei der Lichtwellenleiter (1) und/oder die Halterung (32) beweglich gelagert ist;
Fokussieren von hochenergetischer Laserstrahlung (22) mittels einer Fokussiereinrichtung (24) auf eine Fokuslage, wobei die Fokuslage im Inneren des Lichtwellenleiters (1) positionierbar ist und wobei die hochenergetische Strahlung (22) von einer Laserstrahlquelle (21) im Pulsbetrieb erzeugt wird;
wobei eine lineare Relativbewegung zwischen dem Lichtwellenleiter (1) und der Fokuslage ausschließlich durch eine Bewegung des Lichtwellenleiters (1) erfolgt; und Bewegung der Fokuslage durch das Innere des Lichtwellenleiters (1) durch die Bewegung des Lichtwellenleiters (1) mit einer Rotation des Lichtwellenleiters (1) erfolgt, und die Geschwindigkeit der Rotation nur durch die Bewegung des Lichtwellenleiters (1) bei der Bewegung verändert wird.

12. Verfahren nach Anspruch 11, wobei die Bewegung der Fokuslage durch das Innere des Lichtwellenleiters (1) in Abhängigkeit einer Repetitionsrate gewählt wird.

13. Verfahren nach einem der Ansprüche 11 oder 12, wobei die Fokuslage kontinuierlich durch den Lichtwellenleiter (1) bewegt wird.

14. Verfahren nach einem der Ansprüche 12 oder 13, wobei die Bewegung der Fokuslage durch den Lichtwellenleiter (1) eine Kombination aus Rotationsbewegung (β₂) und Translationsbewegung (Z) ist.

15. Vorrichtung zur Einbringung von Mikromodifikationen (5, 51, 52, 53, 54, 55, 56) in einen Lichtwellenleiter (1), umfassend:
eine Fokussiereinrichtung (24) zur Fokussierung von gepulster Laserstrahlung (22) in den Innenbereich des Lichtwellenleiters (1);
mindestens eine motorisch angetriebene Positioniereinrichtung (31, 33, 34) für eine linearen Bewegung der Fokuslage zum Lichtwellenleiter (1) und , wobei die Bewegung ausschließlich durch Bewegung des Lichtwellenleiters (1) erfolgt;
eine Halterung oder Führung (32) für den Lichtwellenleiter (1) ;
eine Rotationsvorrichtung (20) zur Rotation Drehen des Lichtwellenleiters (1); und
wobei die Vorrichtung so ausgelegt ist, dass die Fokusposition durch das Innere des Lichtwellenleiters (1) mittels der motorischen Positioniereinrichtung bewegt wird, während eine Rotationsgeschwindigkeit der Bewegung des Lichtwellenleiters (1) ausschließlich durch dessen Bewegung verändert wird.
